Europäisches Patentamt

European Patent Office

Office européen des brevets

(11) Publication number: **0 243 015 B1**

## EUROPEAN PATENT SPECIFICATION

(45) Date of publication of patent specification: **11.11.92** (51) Int. Cl.5: **C12Q 1/12**

(21) Application number: **87302806.2**

(22) Date of filing: **31.03.87**

The file contains technical information submitted
after the application was filed and not included in
this specification

(54) **Selective media for growth of Neisseria.**

(30) Priority: **25.04.86 US 855554**

(43) Date of publication of application:
**28.10.87 Bulletin 87/44**

(45) Publication of the grant of the patent:
**11.11.92 Bulletin 92/46**

(84) Designated Contracting States:
**BE CH DE ES FR GB GR IT LI NL SE**

(56) References cited:
**US-A- 3 846 241**

**CHEMICAL ABSTRACTS, vol. 81, no. 25, 23rd
December 1974, page 280, abstract no.
166238f, Columbus, Ohio, US; I. PHILLIPS et
al.: "Diagnosis of gonorrhea by culture on a
selective medium containing vancomycin,
colistin nystatin, and trimethoprim (VCNT).
Comparison with gram-staining and im-
munofluorescence", & BR. J. VENER. DIS.
1972, 48(4), 287-92**

(73) Proprietor: **BECTON, DICKINSON & COMPANY
One Becton Drive
Franklin Lakes New Jersey 07417-1880(US)**

(72) Inventor: **Evans, George L.
10301-1 Malcolm Circle
Cockeysville Maryland(US)**
Inventor: **DeVaux-Pyta, Deborah L.
870 Century Street
Hampstead Maryland 21074(US)**

(74) Representative: **Ruffles, Graham Keith et al
MARKS & CLERK 57-60 Lincoln's Inn Fields
London WC2A 3LS(GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

Rank Xerox (UK) Business Services

BIOLOGICAL ABSTRACTS, vol. 66, no. 4, 1978, page 2182, abstract no. 22286, Philadelphia, PA, US; Y.C. FAUR et al.: "The selectivity of vancomycin and lincomycin in NYC medium for the recovery of N. gonorrhoeae from clinical specimens", & HEALTH LAB. SCI. 15(1): 22-27. 1978

BIOLOGICAL ABSTRACTS, vol. 73, no. 2, 1982, page 1127, abstract no. 10963, Philadelphia, PA, US; S. MIRRETT et al.: "Neisseria gonorrhoeae strains inhibited by vancomycin in selective media and correlation with auxotype", & CLIN. MICROBIOL. 14(1): 94-99. 1981

CHEMICAL ABSTRACTS, vol. 103, no. 23, 9th December 1985, page 397, abstract no. 193055y, Columbus, Ohio, US; M. FUZI: "Selective culturing of penicillinase-producing strains of Neisseria gonorrhoeae", & KISERL. ORVOSTUD. 1985, 37(4), 438-441

**Description**

The present invention relates to rapid detection and presumptive identification of Neisseria species, particularly Neisseria gonorrhoeae, by means of a selective blood agar medium.

BACKGROUND OF THE INVENTION

The need for developing means for rapid and accurate cultivation and primary isolation of pathogenic Neisseria species, such as N. gonorrhoeae and N. meningitidis, has greatly increased in recent years. A substantial amount of research and development has occurred in an attempt to provide a growth medium for Neisseria which is selective for Neisseria and which inhibits the growth of other microorganisms. A principal problem occurs in that antibiotics which are associated with selective media for Neisseria inhibit the growth of antibiotic-sensitive Neisseria species, such as N. gonorrhoeae. Also, antibiotics, such as vancomycin, which are associated with selective media for N. gonorrhoeae, while prohibiting growth of certain sensitive N. gonorrhoeae, also do not act to inhibit growth of other particular gram negative species, such as Capnocytophaga.

The high level of activity and concern for the development of an improved selective media for N. gonorrhoeae is amply demonstrated by the following list of references which are all, in some way, associated with the development of selective media for the isolation and growth of N. gonorrhoeae:

Anstey, R.J.,J. Gun-Munro, R.P. Rennie, J.H. Thornley, D.G. Schaus, R. Lannigan, Z. Hussain, and R.S. Maharaja. 1984. Laboratory and clinical evaluation of modified New York City medium (Henderson formulation) for the isolation of Neisseria gonorrhoeae. J. Clin. Microbiol.20:905-908.

Berger, U. 1966. A new selective medium for Neisseria meningitidis and Neisseria gonorrhoeae. A. med. Mikrobiol. u. Immunol. 152: 169-172.

Bonin, P., T. Tanino, and H.H. Handsfield. 1984. Isolation of Neisseria gonorrhoeae on selective and nonselective media in a sexually transmitted disease clinic. J. Clin. Microbiol. 19: 218-392.

Brorson, J., I. Holmberg, B. Nygren, and S. Seeberg. 1973. Vancomycin-sensitive strains of Neisseria gonorrhoeae: a problem for the diagnostic laboratory. Brit. J. Vener. Dis. 49:452-453.

Cross, R., M.B. Hoger, R. Neibaur, B. Paternack, and F. Brady. 1971. VCN-inhibited strains of Neisseria gonorrhoeae. HSMHA Health Reports. 86: 990-992.

Faur, Y.C., M.H. Weisburd, and M.E. Wilson, 1978. The selectivity of vancomycin and lincomycin in NYC medium for the recovery of N. gonorrhoeae from clinical specimens. Health Lab. Sci. 15: 22-27.

Faur, Y.C., and M.E. Wilson. 1982. Correspondence to the editor re: lincomycin versus vancomycin in New York City (NYC) medium for the cultural diagnosis of gonorrhoeae. Brit. J. Vener. Dis. 58: 66.

Granato, P.A., J.L. Paepke, and L.B. Weiner. 1980. Comparison of modified New York City medium with Martin-Lewis medium for recovery of Neisseria gonorrhoeae from clinical specimens. J. Clin. Microbiol. 12: 748-752.

Hipp, S., W. Lawton, N. Chen, and H. Gaafer, 1974. Inhibition of Neisseria gonorrhoeae by a factor produced by Candida albicans. Appl. Microbiol. 27: 192-196.

Kraus, S., R. Geller, G. Perkins, and D. Rhoden. 1976. Interference of Neisseria gonorrhoeae growth by other bacterial species. J. Clin. Microbiol. 4: 288-295.

Martin, J.E., Jr., S.B. Samuels, W.L. Peacock, Jr., and J.D. Thayer. 1964. Neisseria gonorrhoeae and Neisseria meningitidis sensitivity to spectinomycin, lincomycin, and penicillin G. Antimicrob. Agents Chemo. p. 437-439.

Martin, J.E., Jr., T.E. Billings, J. Hackney, J. Thayer. 1967. primary isolation of N. gonorrhoeae with a new commercial medium. Public Health Reports. 82: 361-363.

Martin, J.E., J. Armstrong, and P.B. Smith. 1971. New system for cultivation of Neisseria gonorrhoeae. Appl. Microbiol. 27: 802-805.

Martin, J.E., Jr., and R. Jackson, 1975. A biological environmental chamber for the culture of Neisseria gonorrhoeae. J. Am. Vener. Dis. Assoc. 2: 28-30.

Mirret, S., L. Reller, and J. Knapp. 1981. Neisseria gonorrhoeae strains inhibited by vancomycin in selective media and correlation with auxotype. J. Clin. Microbiol. 14: 94-99.

Odegaard, K., O. Solberg, J. Lind, G. Myhre, and B. Nyland. 1975. Lincomycin in selective medium for the isolation of Neisseria gonorrhoeae. Acta. path. microbiol. scand. Sect. B. 83: 301-304.

Phillips, I., D. Humphrey, A. Middleton, and C.S. Nicol. 1972. Diagnosis of gonorrhoeae by culture on a selective medium containing vancomycin, colistin, nystatin, and trimethoprim (VCNT). Brit. J. Vener. Dis. 48: 287-292.

Potuznik, V., O. Hausner. 1967. Selective medium with lincomycin and colistin for the isolation of pathogenic Neisseria. J. Hygiene, Cpid., Micro. 11: 127.

Reyn, A. and M. Bentzon. 1972. Comparison of a selective and a nonselective medium in the diagnosis of gonorrhoeae to ascertain the sensitivity of Neisseria gonorrhoeae to vancomycin. Brit. J. Vener. Dis. 48: 363-368.

Robinson, M., C. Hicks, and G. Davidson, 1970. Use of vancomycin, colistimethate, nystatin medium to transport gonococcal specimens. Public Health Reports. 85: 390-392.

Svarva, P., and J. Maeland. 1979. Comparison of two selective media in the cultural diagnosis of gonorrhoeae. Acta. path. microbiol scanda. Sect. B., 87: 391-391.

Symington, D.A. 1975. Improved transport system for Neisseria gonorrhoeae in clinical specimens. J. Clin. Microbiol. 2: 498-503.

Thayer, J.D., and J.E. Martin, Jr. 1966. Improved medium selective for cultivation of N. gonorrhoeae and N. meningitidis. Public Health Reports. 81: 559-562.

Young, H. 1978. Cultural diagnosis of gonorrhoeae with modified New York City (MNYC) medium. Brit. J. Vener. dis. 54: 36-40.

Young, H. 1978. Identification and penicillinase testing of Neisseria gonorrhoeae from primary isolation cultures on modified New York City medium. J. Clin. Microbiol. 7: 247-250.

While the above list of prior art references amply demonstrate the need for an improved growth media for the isolation and detection of Neisseria species, such as N. gonorrhoeae, none of the references set forth herein above describe the present invention which is directed to the provision of a highly selective culture medium for N. gonorrhoeae and N. meningitidis.

## SUMMARY OF THE INVENTION

In general, in accordance with the invention, there is provided a nutrient growth medium which is highly selective for pathogenic Neisseria. The nutrient growth medium includes an antibiotic agent which is a combination of vancomycin and lincomycin. The vancomycin is used at levels substantially lower than heretofore reported for the use of vancomycin in culture media used for the selective culturing of pathogenic Neisseria species, such as N. gonorrhoeae. The use of lower levels of vancomycin permits the growth of vancomycin-sensitive Neisseria species. The lincomycin is used at a sufficient level to inhibit the growth of other gram negative species, such as Capnocytophaga and staphylococci, which have been a problem in the use of other selective media for Neisseria which have used reduced levels of vancomycin as the sole antibiotic. Also present in the selective growth media of the present invention are a diaminopyrimidine, such as trimethoprim, for inhibition of Proteus species, an anti-fungal agent and a polymixin.

Accordingly, it is a primary object of the present invention to provide a growth medium for improved culturing of pathogenic Neisseria.

It is another object of the present invention to provide a medium and method for culturing pathogenic Neisseria which permits primary isolation of pathogenic species of the Neisseria genus.

It is a further object of the present invention to provide a composition which is selective for the growth of pathogenic Neisseria but which inhibits growth of other gram negative species.

## DESCRIPTION OF THE PREFERRED EMBODIMENTS

The components of the selective culture medium of the present invention include a nutrient agar base, a source of hemoglobin, an enrichment agent and selective agents.

The preferred nutrient agar base is GC Agar base which has the following composition in grams per liter of completed medium:

| | |
|---|---|
| Pancreatic digest of casein | 7.5 g |
| Peptic digest of animal tissue | 7.5 |
| Corn starch | 1.0 |
| Dipotassium phosphate | 4.0 |
| Monopotassium phosphate | 1.0 |
| Sodium chloride | 5.0 |
| Agar | 12.0 |

The preferred source of hemoglobin is dried bovine hemoglobin in a concentration of approximately 10.0 g/L.

The preferred enrichment agent is supplied by the BBL Microbiology Systems Division of Becton-Dickinson and Company under the tradename IsoVitaleX® Enrichment. The final concentration of IsoVitaleX® Enrichment is used in the nutrient agar base 10.0 ml per liter. The composition per liter of IsoVitaleX® Enrichment is:

| | |
|---|---|
| Vitamin B12 | 0.01g |
| L-Glutamine | 10.0 |
| Adenine | 1.0 |
| Guanine Hydrochloride | 0.03 |
| p-Aminobenzoic Acid | 0.013 |
| Nicotinamide Adenine Dinucleotide | 0.25 |
| Thiamine Pyrophosphate | 0.1 |
| Ferric Nitrate | 0.02 |
| Thiamine Hydrochloride | 0.003 |
| L-Cysteine Hydrochloride | 25.9 |
| L-Cystine | 1.1 |
| Dextrose | 100.0 |

The inhibitor of the invention includes an antibiotic agent that inhibits gram-positive bacteria, especially staphylococci and is active against Capnocytophaga species, an agent active against gram-negative bacteria, an agent that inhibits Proteus species, and an antifungal agent.

The preferred antibiotic agent for inhibition of gram-positive bacteria and Capnocytophaga is a combination of vancomycin and lincomycin which allows the growth of even vancomycin-sensitive Neisseria gonorrhoeae while completely inhibiting the growth of Capnocytophaga species and Staphylococci including vancomycin-resistant strains. The amount of vancomycin is from about 1.0 to 4.0 micrograms per liter of growth medium and the lincomycin from about 0.5 to about 3.0 micrograms per milliliter of growth medium.

Lincomycin is known for use as a replacement for vancomycin in a selective medium for the isolation of N. gonorrhoeae, K. Odegaard et al, "Lincomycin In Selective Medium For The Isolation of Neisseria Gonorrhoeae", Acta. path. microbiol. scand. Sect. B, 83: 301-304, 1975. However, there is no suggestion in this reference or any other reference that the inventors are aware of that lincomycin can be combined with vancomycin to provide an improved antibiotic agent in a selective medium for the isolation of Neisseria species.

Lincomycin is also known for use as an anti-bacterial agent against the majority of strains of Bacteroides. However, it is known that lincomycin is ineffective against meningococci and gonococci. Clindamycin is a clorinate derivative of lincomycin and is known to be more active in vivo than lincomycin. However, clindamycin cannot replace lincomycin in the selective growth medium of the present invention

5

since it is active against Neisseria species.

The agent active against Proteus species is a diaminopyrimidine. The preferred Proteus active agent is trimethoprim or trimethoprim lactate in a concentration of 2 to about 8 mg per liter

The anti-fungal agent is preferably selected from the group consisting of nystatin, anisomycin, miconazole and amphotericin B. The preferred anti-fungal agent is amphoterian B and is present in the selective growth medium of the present invention at a level of from about 2 to about 8 micrograms per liter of growth medium. The agent active against gram-negative bacteria is preferably a polymyxin which is preferably present in the growth medium at a level from about 5 to about 10 micrograms per liter of growth medium. The polymyxin is preferably colistin sulphate.

The following examples are provided to further illustrate the present invention. The scope of the invention is not, however, meant to be limited to the specific details of the examples.

Example 1

To prepare one liter of the inhibitor of the present invention, each of the following is dissolved in about 750 ml of purified water: (1) 200 milligram vancomycin, (2) 750 milligram colistin sulphate or colistin sodium methane sulfonate, (3) 500 milligram amphotericin B which is first dissolved in a small amount of 1 N.NaOH, (4) 100 milligram lincomycin, and (5) 500 milligram trimethoprim base or trimethoprim lactate. Sufficient purified water is added to the inhibitor solution to bring the volume to 1 liter. The solution is then sterilized by filtration through a 0.22 micron filter.

A similar inhibitor solution is prepared wherein the lincomycin is deleted from the formula, the vancomycin is adjusted to 300 milligram per liter,and the amphotericin B is replaced by nystatin.

A modified Thayer-Martin medium is made by combining GC Agar Base, hemoglobin, IsoVitaleX® Enrichment and the above described inhibitor that does not contain lincomycin. A similar preparation is made using the inhibitor of the present invention as described above containing lincomycin. The two growth media have the composition indicated hereinbelow:

| Ingredient | Growth Medium of the Invention (Medium A) | Thayer-Martin Selective Agar (Medium B) |
|---|---|---|
| GC Agar Base | 36.0 gram/l | 36.0 gram/l |
| Hemoglobin | 10.0 gram/l | 10.0 gram/l |
| IsoVitaleX® Enrichment | 10.0 ml | 10.0 ml |
| Vancomycin | 2.0 mg/l | 3.0 mg/l |
| Lincomycin | 1.0 liter | --- liter |
| Colistin Sulfate | 7.5 liter | 7.5 liter |
| Trimethoprim | 5.0 liter | 5.0 liter |
| Amphotericin B | 5.0 liter | --- |
| Nystatin | --- | 1250 units |

After the growth media are prepared, they are dispensed into 100mm petri dishes according to standard bacteriological practice. After the medium has gelled, the dishes are placed in a suitable container and are stored at 2 to 8°C until ready for use. The plates may be stored at this temperature for up to 14 weeks.

Both stock cultures and clinical specimens were used to evaluate the performance of the two media. The cultures were inoculated according to standard bacteriologic practices; growth or inhibition was scored after appropriate incubation in a CO2-enriched environment.

The ATCC cultures used included Neisseria gonorrhoeae 43069, 43070, 35201, N. meningitidis 13090, Candida albicans 60193, Neisseria sicca 9913, Proteus mirabilis 43071, Staphyloccocus epidermidis 12228, and Capnocytophaga ochracea 33595. Six clinical strains of vancomycin-susceptible N. gonorrhoeae from patient specimens were tested as well as six Capnocytophaga species, isolated from oropharyngeal cultures, and one vancomycin-resistant Staphylococcus.

| Parameters | Results Observed on Medium | |
|---|---|---|
| | A | B |
| Recovery of pathogenic Neisseria | ++++ | ++++ |
| Recovery of vancomycin-susceptible N. gonorrhoeae | +++ | ++ |
| Inhibition of Yeasts | ++++ | +++ |
| Inhibition of Capnocytophaga | ++++ | - |
| Inhibition of vancomycin-resistant S. epidermidis | ++++ | + |
| Inhibition of other normal flora | ++++ | ++++ |

```
++++ Excellent      Media Code
 +++ Good           A = Medium of the present invention
  ++ Fair           B = Modified Thayer - Martin Medium
```

The results, as set forth in the above table, illustrate the surprising improvement of the selective agent of the present invention over selective agents known in the prior art.

**Claims**

1. A culture medium for the selective isolation of pathogenic Neisseria which culture medium comprises an antibiotic composition including a mixture of vancomycin and lincomycin.

2. A culture medium as claimed in claim 1 containing from 1 to 4 micrograms per litre of vancomycin and from 0.5 to 3 micrograms per litre of lincomycin.

3. A culture medium as claimed in claim 1 or claim 2 also containing a diaminopyrimidine.

4. A culture medium as claimed in claim 3 containing from 2 to 8 micrograms per litre of the diaminopyrimidine.

5. A culture medium as claimed in any one of the preceding claims also containing an antifungal agent selected from nyotatin, anisomycin, miconayole and amphotericin B.

6. A culture medium as claimed in claim 5 containing from 2 to 8 micrograms per litre of antifungal agent.

7

7. A culture medium as claimed in any one of the preceding claims also containing a polymyxin.

8. A culture medium as claimed in claim 7 containing from 5 to 10 micrograms per litre of polymyxin.

9. An antibiotic agent for use in the selective cultivation of pathogenic species of Neisseria comprising a combination of vancomycin and lincomycin.

**Patentansprüche**

1. Kulturmedium für die selektive Isolierung von pathogenen Neisseria, wobei dieses Kulturmedium eine antibiotische Zusammensetzung umfaßt, welche eine Mischung von Vancomycin und Lincomycin einschließt.

2. Kulturmedium nach Anspruch 1, enthaltend von 1 bis 4 Mikrogramm pro Liter an Vancomycin und von 0,5 bis 3 Mikrogramm pro Liter an Lincomycin.

3. Kulturmedium nach Anspruch 1 oder Anspruch 2, weiter enthaltend ein Diaminopyrimidin.

4. Kulturmedium nach Anspruch 3, enthaltend von 2 bis 8 Mikrogramm pro Liter des Diaminopyrimidins.

5. Kulturmedium nach einem der vorhergehenden Ansprüche, weiter enthaltend ein Fungizid, ausgewählt aus Nyotatin, Anisomycin, Miconayol und Amphotericin B.

6. Kulturmedium nach Anspruch 5, enthaltend von 2 bis 8 Mikrogramm pro Liter des Fungizids.

7. Kulturmedium nach einem der vorhergehenden Ansprüche, weiter enthaltend ein Polymyxin.

8. Kulturmedium nach Anspruch 7, enthaltend von 5 bis 10 Mikrogramm pro Liter an Polymyxin.

9. Antibiotisches Mittel zur Verwendung bei der selektiven Kultivierung von pathogener Spezies Neisseria, umfassend eine Kombination von Vanomycin und Lincomycin.

**Revendications**

1. Un milieu de culture pour l 'isolement sélectif de Neisseria pathogène, lequel milieu comprend une composition antibiotique qui comprend notamment un mélange de vancomycine et lincomycine.

2. Un milieu de culture tel que revendiqué dans la revendication 1, contenant de 1 à 4 microgrammes par litre de vancomycine et de 0.5 à 3 microgrammes par litre de lincomycine.

3. Un milieu de culture tel que revendiqué dans la revendication 1 ou la revendication 2, contenant également une diaminopyrimidine.

4. Un milieu de culture tel que revendiqué dans la revendication 3 contenant de 2 à 8 microgrammes par litre de la diaminopyrimidine.

5. Un milieu de culture tel que revendiqué dans l'une quelconque des revendications précédentes, contenant également un agent antifongique choisi parmi la nystatine, l'anisomycine, le miconayole et l'amphotéricine B.

6. Un milieu de culture tel que revendiqué dans la revendication 5, contenant de 2 à 8 microgrammes par litre de l'agent antifongique.

7. Un milieu de culture tel que revendiqué dans l'une quelconque des revendications précédentes, contenant également une polymyxine.

8. Un milieu de culture tel que revendiqué dans la revendication 7 contenant de 5 à 10 microgrammes par litre de polymyxine.

9. Un agent antibiotique pour un usage dans la culture sélective d'une espèce pathogène de Neisseria, comprenant une association de vancomycine et de lincomycine.